# EUROPEAN PATENT APPLICATION

(11) **EP 3 639 959 A1**
(43) Date of publication of application: **22.04.2020**
(21) Application number: 19202207.7
(22) Date of filing: 09.10.2019
(51) Int. Cl.: B23K 9/095, A61F 9/06, B23K 9/32

(54) **SYSTEMS AND METHODS INCLUDING A HEAD-MOUNTED DEVICE FOR USE IN ARC WELDING**

(30) Priority: 10.10.2018 US 201816156228
(71) Applicant: Lincoln Global, Inc., Santa Fe Springs, CA 90670 (US)
(72) Inventor: MATTHEWS, William T., Chesterland, OH Ohio 44026 (US)
(74) Representative: Grosse Schumacher Knauer von Hirschhausen

(57) **Abstract**

Embodiments of systems (400, 600) and methods related to the automatic adjustment of auto-darkening filter device parameters in response to settings of a welding system (400, 600) are disclosed. One embodiment includes a head-mounted device (100, 605) having a wireless receiver (130), an auto-darkening device (120, 620), and a selection device (124). The wireless receiver (130) is configured to receive wirelessly transmitted communication signals from an arc welding system (400, 600) which represent a present welding current setting and a present welding mode setting of the arc welding system (400, 600). The selection device (124) is configured to automatically select a shade and a sensitivity of the auto-darkening device (120, 620) in response to the present welding current setting and the present welding mode setting. The auto-darkening device (120, 620) is configured to sense the presence of an arc (A), transition from an un-darkened state to a darkened state in response to the arc (A), and transition from the darkened state to the un-darkened state when the arc (A) is no longer present.

## Description

### FIELD OF THE INVENTION

The invention is related to a head-mounted device for use in arc welding according to claim 1 and to an arc welding system according to claims 8 and 15. Embodiments of the present invention relate to head-mounted devices used in arc welding and associated arc welding systems and methods.

### TECHNICAL BACKGROUND

Head-mounted devices such as welding helmets are used in arc welding to protect a user from the dangerous sparks, spatter, and light produced by an arc welding process. A welding helmet typically includes a filter lens which filters out dangerous light produced by the arc welding process to protect the eyes of the user. Some welding helmets include an auto-darkening filter (ADF) which switches between a darkened state and an un-darkened (or less darkened) state in response to the presence or absence of a welding arc. Some welding helmets provide the ability to manually adjust a shade (an amount of darkness or filtering) of the auto-darkening filter.

A welding helmet is one of the most important pieces of personal protective equipment a welder can have. A good helmet protects the eyes and skin not only from severe sparks but also from potentially vision-damaging ultraviolet and infrared rays emitted by the arc. A helmet's protective features, combined with comfort considerations are what welders should consider when selecting the right helmet for their needs.

The right helmet must be able to worn easily and comfortably for a full day's work, providing flexible adjustments, while protecting the eyes and face from spatter and sparks and harmful light rays. Today's helmets are considerably more functional than those of even 10 or 15 years ago. They are designed to accommodate a welder's specific needs on any job. Helmets should meet strict safety standards across the globe. In the United States, that standard is ANSI Z87.1 and in Canada it is CAN/CSA Z94.3, for example. These standards address such concerns as light leakage and flame and impact resistance.

Some welders, particularly many professional pipe welders, still opt to wear traditional welding helmets with a traditional glass lens and fixed shade, which remains darkened at all times. While these helmets do provide rugged and inexpensive safety protection, they also have a few disadvantages. Welding helmets featuring a fixed shade can be more difficult to use because a welder has to lift the helmet every time he or she wants to examine the weldment and joint, set his position and prepare for welding and then flip the helmet down again when it's time to strike the arc. This repetitive movement can cause neck strain and fatigue after a full day's work. Additionally, in tight or restricted spaces, it can be difficult to move the helmet up or down. Also, for less-experienced welders, it can be difficult to keep the MIG gun, TIG torch, or stick electrode in the correct position to begin welding in the joint after the helmet is lowered into place. Poor weld starts can result in weld defects, something any welder wants to avoid.

Serious professional welders may use more advanced auto-darkening helmets with variable controls that adjust the shade from a light state to a dark state and back. These helmets protect from harmful light emissions at all times and darken to almost any pre-selected shade in milliseconds, thanks to quick-changing LCD (Liquid Crystal Display) technology in the auto-darkening cartridges. With auto-darkening helmets, welders can see clearly while the helmet is already in a down position, so that setting up to weld in a weldment joint can be done with the hood in position. These helmets permit more continuous work, reducing unnecessary stop-and-start time and the need for a welder to readjust a helmet and set up positioning.

### DESCRIPTION

In order to improve welder's protection when welding, a head-mounted device for use in arc welding according to claim 1 and an arc welding system according to claims 8 and 15 are described. Preferred embodiments are subject of the sub claims. Embodiments of the present invention include systems and methods related to the automatic adjustment of auto-darkening filter device parameters in response to settings of a welding system, or other related systems.

One embodiment includes a head-mounted device for use in arc welding. The head-mounted device includes a wireless receiver configured to receive wirelessly transmitted communication signals from an arc welding system. The wirelessly transmitted communication signals represent at least a present welding current setting and a present welding mode setting of the arc welding system. The head-mounted device also includes an auto-darkening device. The auto-darkening device is configured to sense a presence of an arc produced by the arc welding system (when an arc is formed, for example, between an electrode and a workpiece), transition from an un-darkened state to a darkened state upon initially sensing the presence of the arc, remain in the darkened state while the arc is present, and transition from the darkened state back to the un-darkened state when the arc is no longer present (i.e., when the arc extinguishes). The head-mounted device further includes a selection device configured to automatically select at least a shade and a sensitivity of the auto-darkening device for transitioning to the darkened state in response to at least the present welding current setting and the present welding mode setting of the arc welding system provided to the head-mounted device via the wirelessly transmitted communication signals received by the wireless receiver. In one embodiment, the selection device is configured as a programmable look-up-table (LUT) to map input signals or input data to output signals or output data. The input signals or input data are derived from the wirelessly transmitted communication signals and represent the present welding current setting and the present welding mode setting. The output signals represent at least the shade and the sensitivity to be automatically selected. In one embodiment, the selection device is further configured to automatically select at least a reaction time of the auto-darkening device for transitioning to the darkened state in response to at least the present welding current setting and the present welding mode setting of the arc welding system provided to the head-mounted device via the wirelessly transmitted communication signals received by the wireless receiver. In one embodiment, the selection device is further configured to automatically select at least a transition delay time of the auto-darkening device for transitioning back to the un-darkened state in response to at least the present welding current setting and the present welding mode setting of the arc welding system provided to the head-mounted device via the wirelessly transmitted communication signals received by the wireless receiver. The wirelessly transmitted communication signals may include, for example, at least one of Wi-Fi® radio frequency spectrum signals, BLUETOOTH® radio frequency spectrum signals, ZIGBEE® radio frequency spectrum signals, infrared spectrum signals, visual spectrum signals, ultrasonic spectrum signals, and audible spectrum signals. A welding mode of the present welding mode setting may include, for example, one of a gas metal arc welding (GMAW) mode, a flux-cored arc welding (FCAW) mode, a gas tungsten arc welding (GTAW) mode, and a shielded metal arc welding (SMAW) mode. In one embodiment, the selection device is integrated into the wireless receiver, and the wireless receiver is operationally connected to the auto-darkening device. In another embodiment, the selection device is integrated into the auto-darkening device and the wireless receiver is operationally connected to the auto-darkening device.

One embodiment includes an arc welding system. The arc welding system includes a welding power supply having a controller and a wireless transmitter. The wireless transmitter is configured to wirelessly transmit communication signals representing at least a present welding current setting and a present welding mode setting of the welding power supply received from the controller. The arc welding system also includes a head-mounted device. The head-mounted device includes a wireless receiver configured to wirelessly receive the communication signals transmitted by the wireless transmitter. The head-mounted device also includes an auto-darkening device. The auto-darkening device is configured to sense a presence of an arc produced by the arc welding system (when an arc is formed, for example, between an electrode and a workpiece), transition from an un-darkened state to a darkened state upon initially sensing the presence of the arc, remain in the darkened state while the arc is present, and transition from the darkened state back to the un-darkened state when the arc is no longer present (i.e., when the arc extinguishes). The head-mounted device further includes a selection device configured to automatically select at least a shade and a sensitivity of the auto-darkening device for transitioning to the darkened state in response to at least the present welding current setting and the present welding mode setting of the welding power supply provided to the head-mounted device via the communication signals received by the wireless receiver. In one embodiment, the selection device is configured as a programmable look-up-table (LUT) to map input signals or input data to output signals or output data. The input signals or input data are derived from the communication signals and represent the present welding current setting and the present welding mode setting. The output signals or output data represent at least the shade and the sensitivity to be automatically selected. In one embodiment, the selection device is further configured to automatically select at least a reaction time of the auto-darkening device for transitioning to the darkened state in response to at least the present welding current setting and the present welding mode setting of the welding power supply provided to the head-mounted device via the communication signals wirelessly received by the wireless receiver. In one embodiment, the selection device is further configured to automatically select at least a transition delay time of the auto-darkening device for transitioning back to the un-darkened state in response to at least the present welding current setting and the present welding mode setting of the welding power supply provided to the head-mounted device via the communication signals wirelessly received by the wireless receiver. The communication signals may include, for example, at least one of Wi-Fi® radio frequency spectrum signals, BLUETOOTH® radio frequency spectrum signals, ZIGBEE® radio frequency spectrum signals, infrared spectrum signals, visual spectrum signals, ultrasonic spectrum signals, and audible spectrum signals. A welding mode of the present welding mode setting may include, for example, one of a gas metal arc welding (GMAW) mode, a flux-cored arc welding (FCAW) mode, a gas tungsten arc welding (GTAW) mode, and a shielded metal arc welding (SMAW) mode. In one embodiment, the selection device is integrated into the wireless receiver, and the wireless receiver is operationally connected to the auto-darkening device. In another embodiment, the selection device is integrated into the auto-darkening device and the wireless receiver is operationally connected to the auto-darkening device.

One embodiment includes an arc welding system. The arc welding system includes a head-mounted device including an auto-darkening device. The auto-darkening device is configured to sense the presence of an arc produced by the arc welding system, transition from an un-darkened state to a darkened state upon initially sensing the presence of the arc, remain in the darkened state while the arc is present, and transition from the darkened state back to the un-darkened state when the arc is no longer present. The arc welding system also includes a welding power supply having a controller, having a selection device, and a wireless transmitter. The selection device is configured to map input signals or input data, representing a present welding current setting and a present welding mode setting of the arc welding system, to output signals or output data, representing at least an automatically selected shade and sensitivity for the auto-darkening device. The wireless transmitter is configured to wirelessly transmit communication signals representing at least the automatically selected shade and sensitivity. The head-mounted device further includes a wireless receiver configured to wirelessly receive the communication signals transmitted by the wireless transmitter to automatically set at least the automatically selected shade and sensitivity in the auto-darkening device for transitioning to the darkened state. In one embodiment, the selection device is configured as a programmable look-up-table (LUT) and includes at least one of an electrically erasable programmable read only memory (EEPROM) device, a field programmable gate array (FPGA) device, a random access memory (RAM) device, and a microprocessor device. In one embodiment, the selection device is further configured to map the input signals or the input data, representing the present welding current setting and the present welding mode setting of the arc welding system, to additional output signals or output data, representing at least an automatically selected reaction time for the auto-darkening device for transitioning to the darkened state. In one embodiment, the selection device is further configured to map the input signals or the input data, representing the present welding current setting and the present welding mode setting of the arc welding system, to additional output signals or output data, representing at least an automatically selected transition delay time for the auto-darkening device for transitioning back to the un-darkened state.

Numerous aspects of the general inventive concepts will become readily apparent from the following detailed description of exemplary embodiments, from the claims, and from the accompanying drawings.

### BRIEF DESCRIPTION OF THE DRAWINGS

The accompanying drawings, which are incorporated in and constitute a part of the specification, illustrate various embodiments of the disclosure. It will be appreciated that the illustrated element boundaries (e.g., boxes, groups of boxes, or other shapes) in the figures represent one embodiment of boundaries. In some embodiments, one element may be designed as multiple elements or multiple elements may be designed as one element. In some embodiments, an element shown as an internal component of another element may be implemented as an external component and vice versa. Furthermore, elements may not be drawn to scale.
FIG. 1 illustrates one embodiment of a head-mounted device for use in arc welding;
FIG. 2 illustrates one embodiment of an auto-darkening device of the head-mounted device of FIG. 1;
FIG. 3 illustrates one embodiment of a look-up-table (LUT) of the auto-darkening device of FIG. 2;
FIG. 4 illustrates one embodiment of an arc welding system having the head-mounted device of FIG. 1;
FIG. 5 illustrates a flowchart of one embodiment of a method performed by the arc welding system of FIG. 5;
FIG. 6 illustrates another embodiment of an arc welding system having another embodiment of a head-mounted device;
FIG. 7 illustrates a flowchart of one embodiment of a method performed by the arc welding system of FIG. 6; and
FIG. 8 illustrates an example embodiment of a controller (e.g., a controller of a welding power supply used in the systems described herein).

### DETAILED DESCRIPTION

Embodiments of systems and methods for automatically adjusting parameters of an auto-darkening device (a.k.a. an auto-darkening filter or ADF) in a head-mounted device are disclosed. For example, one or more of a shade, a sensitivity, a reaction time, or a transition delay time of an auto-darkening device may be adjusted in response to a present welding current setting and/or a present welding mode setting of an arc welding system.

One embodiment includes a head-mounted device having a wireless receiver, an auto-darkening device, and a selection device. The wireless receiver is configured to receive wirelessly transmitted communication signals from an arc welding system. The wirelessly transmitted communication signals represent at least a present welding current setting and a present welding mode setting of the arc welding system. The auto-darkening device is configured to sense a presence of an arc produced by the arc welding system (when an arc is formed, for example, between an electrode and a workpiece), transition from an un-darkened state to a darkened state upon initially sensing the presence of the arc, remain in the darkened state while the arc is present, and transition from the darkened state to the un-darkened state when the arc is no longer present (i.e., when the arc extinguishes). The selection device is configured to automatically select at least a shade and a sensitivity of the auto-darkening device for transitioning to the darkened state in response to at least the present welding current setting and the present welding mode setting of the arc welding system provided to the head-mounted device via the wirelessly transmitted communication signals received by the wireless receiver.

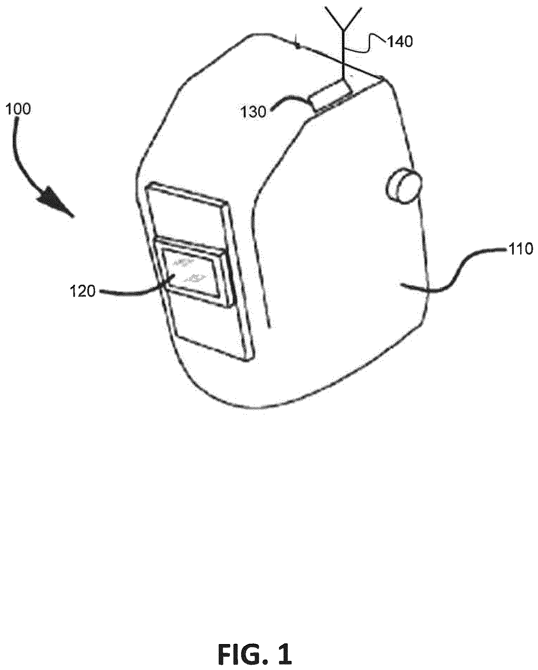

The examples and figures herein are illustrative only and are not meant to limit the subject invention, which is measured by the scope and spirit of the claims. Referring now to the drawings, wherein the showings are for the purpose of illustrating exemplary embodiments of the subject invention only and not for the purpose of limiting same, FIG. 1 illustrates one embodiment of a head-mounted device 100 for use in arc welding using an arc welding system. The head-mounted device 100 is discussed in detail herein with respect to being used for arc welding with an arc welding system. The welding system may be a robotic welding system, a non-robotic welding system (e.g., semi-automatic or manual), or some combination thereof. It is envisioned that a welding system may be used to weld a workpiece by a process such as, for example, gas metal arc welding (GMAW), flux-cored arc welding (FCAW), or gas tungsten arc welding (GTAW). Other processes for welding are possible as well, in accordance with other embodiments. However, in accordance with other embodiments, a head-mounted device may be configured to be used in a similar manner with respect to other systems (e.g., cutting systems or grinding systems) that allow a user to select one or more system parameters such as, for example, a mode setting or an electrical current setting.

With reference to FIG. 1, the head-mounted device 100 includes a protective shell 110, an auto-darkening device 120, and a wireless receiver 130 with an antenna 140. In one embodiment, the auto-darkening device 120 includes one or more batteries (not shown) for powering the various elements of the auto-darkening device 120. The batteries may be, for example, lithium ion batteries. In accordance with one embodiment, parameters of the auto-darkening device 120 are able to be automatically selected or adjusted based on settings of an arc welding system. Such parameters of the auto-darkening device 120 may include one or more of, for example, a shade, a sensitivity, a reaction time, and a transition delay time.

The shade parameter of the auto-darkening device 120 determines how much light (e.g., light produced by an arc) is blocked or filtered to protect the eyes of the user during a welding operation. In one embodiment, the auto-darkening device 120 includes an electronic, auto-darkening LCD cartridge. Auto-darkening LCD cartridges have arc sensors that respond to the light given off by an electric arc during arc welding. The arc sensors control the operation of a liquid crystal display (LCD) lens in the cartridge. The LCD lens can quickly change from a light state, in which a workpiece is readily visible, to a dark state, based on the presence of an arc. When the LCD lens is in the dark state, the operator is protected from the harmful light of the arc.

A welding shade number (level) refers to the ability to filter light. In accordance with one embodiment, the head-mounted device 100 meets the ANSI Z87.1 standard and provides 100% protection against harmful infrared and UV rays. Shade numbers (e.g., for a darkened state) may range from a #8 shade for low current arc welding applications up to a #13 shade for high current arc welding applications, in accordance with one embodiment. Additional ranges (e.g., #3 to #7) may be provided for grinding or cutting applications. In an un-darkened (inactive) state, an auto-darkening device may use a #3 or a #4 shade, which is relatively easy to see through.

The sensitivity parameter of the auto-darkening device 120 refers to how much light or brightness will trigger the lens of the auto-darkening device 120 to switch to a darkened state. The reaction time parameter of the auto-darkening device 120 refers to how fast the lens of the auto-darkening device 120 will switch from the un-darkened state (e.g., shade #3) to the darkened state (e.g., shade # 10). For example, when sensors of the auto-darkening device 120 sense the initiation of an arc, the lens darkens in a fraction of a second (e.g., within 1/12,000 to 1/20,000 of a second), to shade #8 to #13, for example. Faster switching helps to better protect the eyes of the user and reduces the chances of developing eye fatigue, for example, after many arc initiations. The transition delay time parameter refers to how long the auto-darkening device 120 will stay in the darkened state after the arc is extinguished. For example, a short transition delay time may be appropriate for performing many tack welds. A long transition delay time may be appropriate for welding at high current levels.

Traditional auto-darkening devices can include operator controls to manually adjust parameters such as shade, sensitivity, and delay. An operator may desire a higher shade level when the electric arc used for welding is particularly bright, such as when welding thick materials at high current levels. A lower shade level may be desired when using a less intense arc, such as when welding thinner materials at lower current levels. An operator may desire to reduce the sensitivity setting to avoid nuisance switching of the auto-darkening device, such as while working in the presence of other welding operators. Delay controls can be used to lengthen or shorten the amount of time it takes for the lens to return to the un-darkened state following the completion of a weld.

In accordance with one embodiment, one or more of the parameters of the auto-darkening device 120 (shade, sensitivity, reaction time, transition delay time) are automatically selectable based on a present welding current setting and/or a present welding mode setting of an arc welding system, as discussed in more detail later herein. Also, in accordance with one embodiment, manual selection of one or more of the auto-darkening device parameters by a user may be available as well, in addition to the automatic selection. For example, in one embodiment, a user may be able to manually override an automatic selection of a parameter.

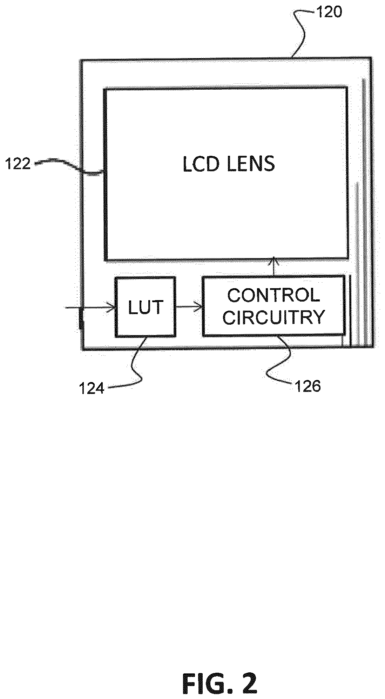

FIG. 2 illustrates one embodiment of the auto-darkening device 120 of the head-mounted device 100 of FIG. 1. The auto-darkening device 120 includes a liquid crystal display (LCD) lens 122 (e.g., as discussed above herein), a selection device 124 configured as a programmable look-up-table (LUT), and control circuitry 126. The wireless receiver 130 of the auto-darkening device 120 is configured to receive wirelessly transmitted communication signals from an arc welding system. The wirelessly transmitted communication signals may include at least one of Wi-Fi® radio frequency spectrum signals, BLUETOOTH® radio frequency spectrum signals, ZIGBEE® radio frequency spectrum signals, visible spectrum signals, infrared spectrum signals, audible spectrum signals, or ultrasonic spectrum signals. Other types of wirelessly transmitted communication signals are possible as well, in accordance with other embodiments.

The wirelessly transmitted communication signals represent at least a present welding current setting and a present welding mode setting of the arc welding system. In accordance with one embodiment, the wireless receiver 130 extracts information from the wirelessly transmitted communication signals upon reception (i.e., information representing the present welding current setting and the present welding mode setting) and provides input signals or input data representing the present welding current setting and the present welding mode setting to the selection device (LUT) 124.

The present welding mode setting may be indicative of, for example, a gas metal arc welding (GMAW) mode, a flux-cored arc welding (FCAW) mode, a gas tungsten arc welding (GTAW) mode, and a shielded metal arc welding (SMAW) mode. The present welding current setting may correspond to, for example, settings between 60 to 80 amps for light GMAW or FCAW welding, settings between 300 to 400 amps for heavy GMAW or FCAW welding, settings between 1-2 amps for light GTAW welding, settings between 200-350 amps for heavy GTAW welding, and settings between 60-300 amps for SMAW welding.

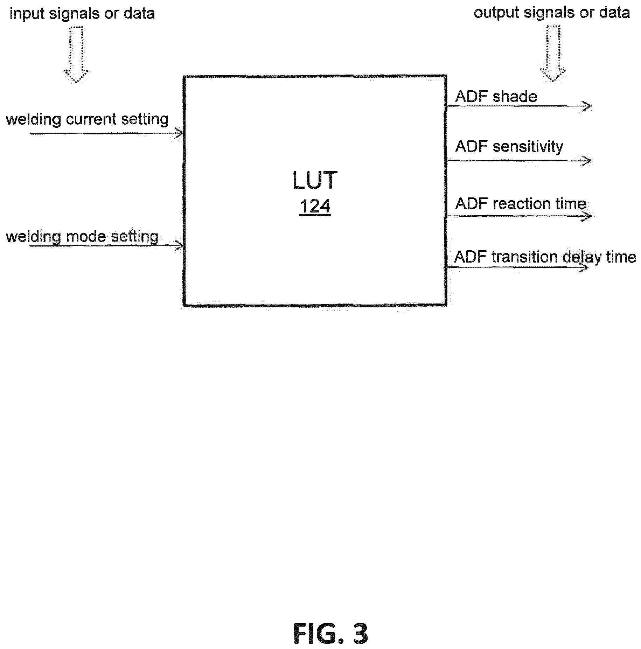

FIG. 3 illustrates one embodiment of the selection device (LUT) 124 of the auto-darkening device 120 of FIG. 2. The signals or data representing the welding current setting and the welding mode setting are provided as inputs to the LUT 124. The LUT 124 is programmed to map the welding current setting and the welding mode setting to a shade value, a sensitivity value, a reaction time value, and a transition delay time value represented as output signals or output data from the LUT 124. That is, as the welding current setting and/or the welding mode setting are changed at the arc welding system, the setting of one or more of the shade, the sensitivity, the reaction time, or the transition delay time may automatically change at the head-mounted device 120, depending on how the LUT 124 is programmed. The LUT 124 may include, for example, at least one of an electrically erasable programmable read only memory (EEPROM) device, a field programmable gate array (FPGA) device, a random access memory (RAM) device, and a microprocessor device. Other types of LUT's are possible as well, in accordance with other embodiments.

The output signals or output data from the LUT 124 are used by the auto-darkening device 120 to set one or more of the shade value, the sensitivity value, the reaction time value, or the transition delay time value to actually be used by the auto-darkening device 120 for the present welding current setting and the present welding mode setting during a welding operation. For example, referring to FIG. 2, in one embodiment the LUT 124 electronically interfaces to the control circuitry 126 of an LCD cartridge of the auto-darkening device 120 to actively and automatically change one or more of the shade, the sensitivity, the reaction time, or the transition delay time of the auto-darkening device 120. In one embodiment, the selection device (LUT) 124 is part of the control circuitry 126. In an alternative embodiment, the selection device (LUT) 124 is integrated into the wireless receiver 130 and the output of the selection device (LUT) 124 in the wireless receiver 130 electronically interfaces to the control circuitry 126 within the auto-darkening device 120. In yet another embodiment, the selection device (LUT) 124 is separate from and interfaces between both the wireless receiver 130 and the auto-darkening device 120.

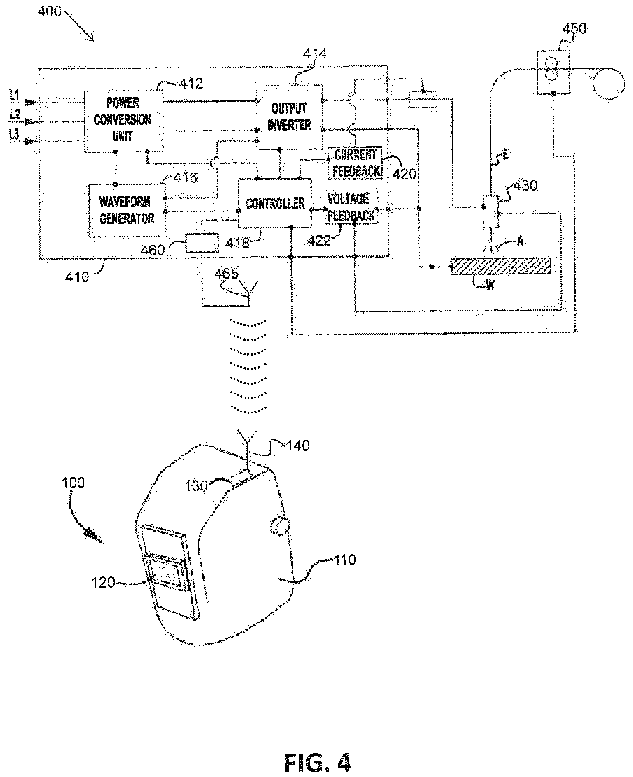

FIG. 4 illustrates one embodiment of an arc welding system 400 having the head-mounted device 100 of FIG. 1. The welding system 400 includes a welding power supply 410 which delivers a welding waveform to a welding torch 430 and a workpiece W through an electrode E to generate a welding arc A. The electrode E is delivered to the welding operation via a wire feeder 450. The wire feeder 450 can be of any known construction such that it is capable of delivering the electrode E to the weld and, in some embodiments, the wire feeder 450 can adjust the wire feed speed of the electrode E based on a signal from the power supply 410.

The general construction of the power supply 410 can be similar to that of known power supplies that are capable of, for example, GMAW/MIG type welding operations, so long as the power supply 410 is capable of functioning and operating as described herein. For example, the power supply 410 can be constructed similar to that of the Power Wave® type power supplies, manufactured by The Lincoln Electric Company, of Cleveland, Ohio. Of course, embodiments of the present invention are not limited to such a construction, and this is intended to be merely exemplary.

As shown in FIG. 4, the power supply 410 is configured to receive an input signal through L1, L2 and L3. FIG. 4 depicts a 3-phase input, but other embodiments can utilize a single phase input. The power supply 410 includes a power conversion unit 412 which is capable of receiving the input signal and outputting a signal to an output phase (such as output inverter 414) so that the output of the power supply 410 is capable of sustaining a welding arc. The power conversion unit 412 can be made up of a number of different components. For example, it can be comprised of a rectifier circuit and a buck-boost circuit which receives the rectified signal and outputs a constant voltage to the output inverter 414. Of course in other exemplary embodiments, the output inverter 414 can be a chopper, or any other type of output circuit that is capable of working with the power conversion unit 412 to output a welding signal.

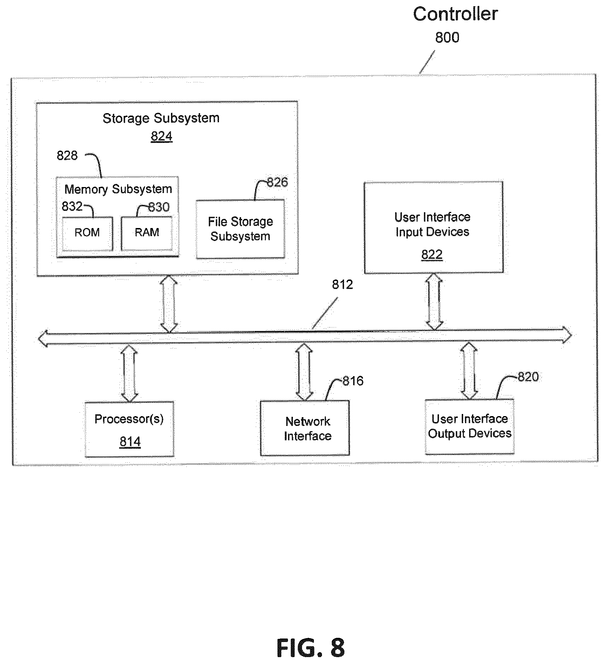

The power supply 410 also includes a waveform generator 416 which is a circuit which aids in controlling the output of at least one, or both, of the power conversion unit 412 and the output inverter 414 to provide the desired welding waveform to be used to generate the arc A. For example, the waveform generator 416 can be used to generate a desired current waveform used to create and maintain the arc A during welding, coupled with one or both of the power conversion unit 412 and the output inverter 414 (or whatever output component is utilized). In addition, the power supply has a controller 418 which can be, for example, any type of CPU or processor-type device capable of controlling functions and operations of the power supply 410. Such controllers are generally known (e.g., see the controller 800 of FIG. 8 herein). Other types of controllers are possible as well having, for example, various types of logic circuitry and memory.

In one embodiment, the controller receives feedback from a current feedback circuit 420 and a voltage feedback circuit 422 which provide current and voltage feedback (respectively) from the welding arc A during a welding operation. With this feedback, the controller 418 is able to adjust and optimize the performance of the power supply 410 to provide the desired output. As shown in FIG. 4, in some embodiments, the controller 418 is also coupled to a wire feeder 450 which allows the controller to receive feedback from the wire feeder 450 as well as control the operation of the wire feeder 450, such as wire feed speed, during a welding operation.

In FIG. 4, the power supply 410 also includes a wireless transmitter 460 coupled to an antenna 465. The wireless transmitter 460 is configured to wirelessly transmit communication signals representing, for example, a present welding current setting and a present welding mode setting of the welding power supply received from the controller 418. For example, the controller 418 may include or be coupled to a user interface (e.g., see the user interface input devices 822 of FIG. 8) which allows a user to select at least a welding current setting and a welding mode setting.

The wireless receiver 130 of the head-mounted device 100 is configured to wirelessly receive the communication signals transmitted by the wireless transmitter 460 of the power supply 410 of the welding system 400. In this manner, auto-darkening parameters of the head-mounted device 100 can be automatically selected and set based on at least the present welding current setting and the present welding mode setting of the welding system 400 as described herein. Again, the communication signals may be in the form of, for example, one or more of Wi-Fi® radio frequency spectrum signals, BLUETOOTH® radio frequency spectrum signals, ZIGBEE® radio frequency spectrum signals, infrared spectrum signals, visual spectrum signals, ultrasonic spectrum signals, and audible spectrum signals.

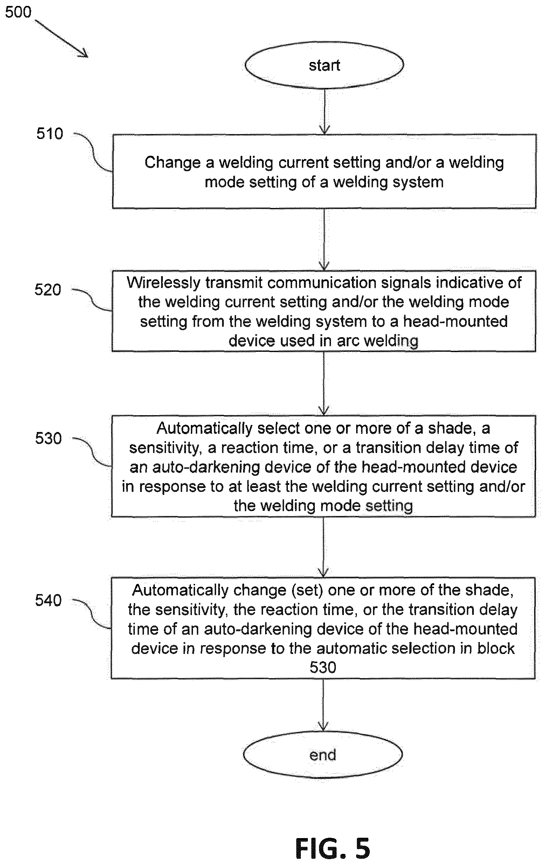

FIG. 5 illustrates a flowchart of one embodiment of a method 500 performed by the arc welding system 400 of FIG. 5. At block 510 of the method 500, a welding current setting and/or a welding mode setting of an arc welding system (e.g., the arc welding system 400 of FIG. 4) is changed (e.g., by a user). At block 520, communication signals indicative of the present welding current setting and/or the present welding mode setting are wirelessly transmitted from the arc welding system (e.g., from the power supply 410 of the arc welding system 400 of FIG. 4) to a head-mounted device (e.g., the head-mounted device 100 of FIG. 4) used in an arc welding process. At block 530, one or more of a shade, a sensitivity, a reaction time, or a transition delay time of an auto-darkening device (e.g. the auto-darkening device 120 of FIG. 2) of the head-mounted device is automatically selected (e.g., via the selection device 124 of FIG. 2 and FIG. 3) in response to the welding current setting and/or the welding mode setting. At block 540, one or more of the shade, the sensitivity, the reaction time, or the transition delay time of the auto-darkening device is automatically changed or set (e.g., by the control circuitry 126 of FIG. 2) in response to the automatic selection in block 530.

In this manner, the automatic mapping of a present welding current setting and/or a present welding mode setting to one or more parameters of an auto-darkening device of a head-mounted device can be performed by the head-mounted device (e.g., as part of an LCD cartridge of the head-mounted device) without the user having to manually adjust the parameters of the head-mounted device. Programming of the selection device (LUT) 124 can be performed based on, for example, preferences of a particular user or statistical data representing the preferences across many users.

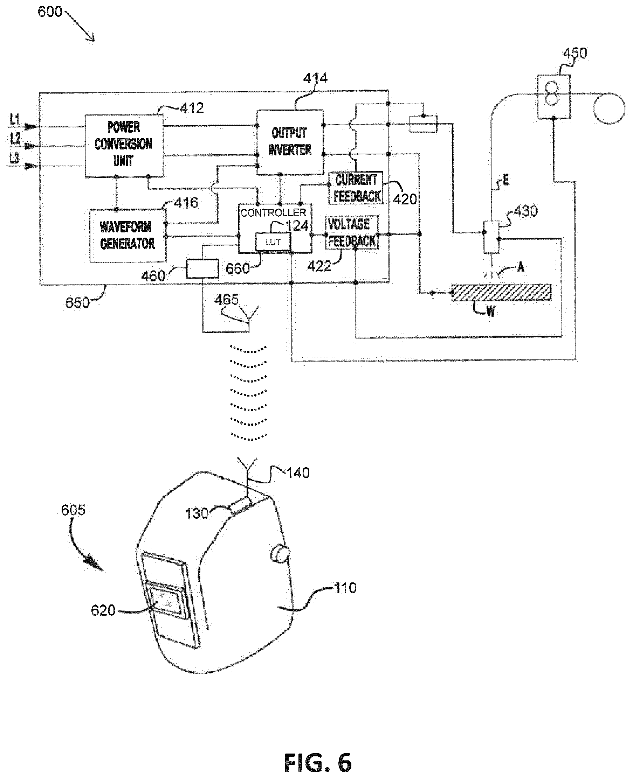

FIG. 6 illustrates another embodiment of an arc welding system 600 having another embodiment of a head-mounted device 605. The arc welding system 600 is similar to the arc welding system 400 of FIG. 4 except that the selection device (LUT) 124 is in a controller 660 of a welding power supply 650 instead of in a head-mounted device. That is, the head-mounted device 605 includes an auto-darkening device 620, which is similar to the auto-darkening device 120 of FIG. 2, but which does not have the selection device (LUT) 124.

In this manner, the selection (of the shade, the sensitivity, the reaction time, and the transition delay time) occurs in the welding power supply 650 in response to a present welding current setting and/or a present welding mode setting. Information indicative of one or more of the shade, the sensitivity, the reaction time, or the transition delay time (instead of the present welding current setting and the present welding mode) is wirelessly transmitted as communication signals by the wireless transmitter 460 of the power supply 650 to the wireless receiver 130 of the head-mounted device 605. The wireless receiver 130 electronically interfaces to the auto-darkening device 620 (which does not have the LUT 124 but has the control circuitry 126) to automatically change (set) one or more of the shade, the sensitivity, the reaction time, or the transition delay time of the auto-darkening device 620.

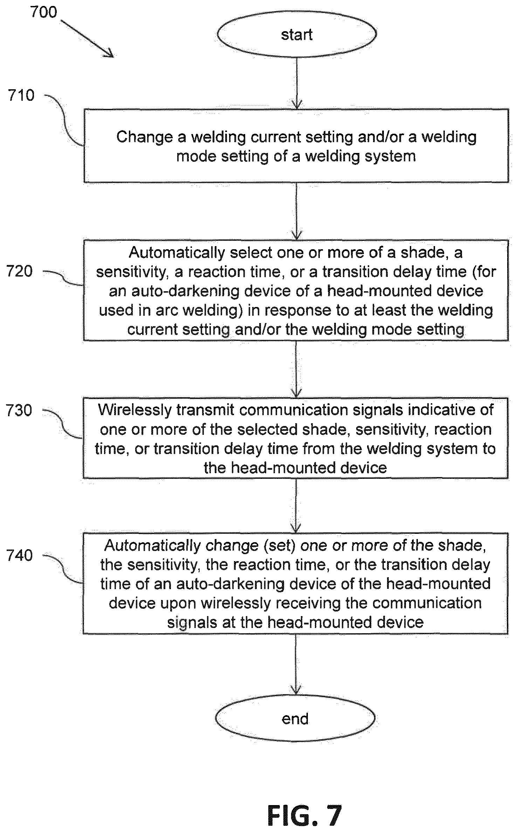

FIG. 7 illustrates a flowchart of one embodiment of a method 700 performed by the arc welding system 600 of FIG. 6. At block 710 of the method 700, a welding current setting and/or a welding mode setting of an arc welding system (e.g., arc welding system 600 of FIG. 6) is changed (e.g., by a user). At block 720, one or more of a shade, a sensitivity, a reaction time, or a transition delay time for an auto-darkening device (e.g. the auto-darkening device 620 of FIG. 6 of the head-mounted device 605) is automatically selected (e.g., via the selection device 124 of FIG. 3 in the controller 660 of the power supply 650) in response to the welding current setting and/or the welding mode setting. At block 730, communication signals indicative of one or more of the shade, the sensitivity, the reaction time, and the transition delay time are wirelessly transmitted from the arc welding system (e.g., from the power supply 650 of the arc welding system 600 of FIG. 6 via the wireless transmitter 460) to a head-mounted device (e.g., the head-mounted device 605 of FIG. 6) used in an arc welding process. At block 740, one or more of the shade, the sensitivity, the reaction time, or the transition delay time of the auto-darkening device (e.g., the auto-darkening device 620) is automatically changed or set upon wirelessly receiving (e.g., via the wireless receiver 130) the transmitted communication signals at the head-mounted device.

In this manner, the automatic mapping of a present welding current setting and/or a present welding mode setting to one or more parameters of an auto-darkening device of a head-mounted device can be performed by a welding power supply (e.g., as part of a controller of a welding power supply) without the user having to manually adjust the parameters of the head-mounted device. Again, programming of the selection device (LUT) 124 can be performed based on, for example, preferences of a particular user or statistical data representing the preferences across many users.

FIG. 8 illustrates an example embodiment of a controller 800. One or more elements of the controller 800 may be used to configure, for example, the control circuitry 126 of FIG. 2, the controller 418 of FIG. 4, or the controller 660 of FIG. 6 used in the systems described herein. The controller 800 includes at least one processor 814 which communicates with a number of peripheral devices via bus subsystem 812. These peripheral devices may include a storage subsystem 824, including, for example, a memory subsystem 828 and a file storage subsystem 826, user interface input devices 822, user interface output devices 820, and a network interface subsystem 816. The input and output devices allow user interaction with the controller 800. Network interface subsystem 816 provides an interface to outside networks and is coupled to corresponding interface devices in other computer systems. For example, the controller 418 of the welding power supply 410 of FIG. 4 may share one or more characteristics with the controller 800 and may be, for example, a conventional computer, a digital signal processor, and/or other computing device.

User interface input devices 822 may include a keyboard, pointing devices such as a mouse, trackball, touchpad, or graphics tablet, a scanner, a touchscreen incorporated into the display, audio input devices such as voice recognition systems, microphones, and/or other types of input devices. In general, use of the term "input device" is intended to include all possible types of devices and ways to input information (e.g., a user selected welding current setting and a user selected welding mode setting) into the controller 800 or onto a communication network.

User interface output devices 820 may include a display subsystem, a printer, a fax machine, or non-visual displays such as audio output devices. The display subsystem may include a cathode ray tube (CRT), a flat-panel device such as a liquid crystal display (LCD), a projection device, or some other mechanism for creating a visible image. The display subsystem may also provide non-visual display such as via audio output devices. In general, use of the term "output device" is intended to include all possible types of devices and ways to output information from the controller 800 to the user or to another machine or computer system.

Storage subsystem 824 stores programming and data constructs that provide or support some or all of the functionality described herein (e.g., as software modules). For example, the storage subsystem 824 may include software modules that are used in a controller to control the welding system 400 of FIG. 4 or the welding system 650 of FIG. 6.

Software modules are generally executed by processor 814 alone or in combination with other processors. Memory 828 used in the storage subsystem can include a number of memories including a main random access memory (RAM) 830 for storage of instructions and data during program execution and a read only memory (ROM) 832 in which fixed instructions are stored. A file storage subsystem 826 can provide persistent storage for program and data files, and may include a hard disk drive, a floppy disk drive along with associated removable media, a CD-ROM drive, an optical drive, or removable media cartridges. The modules implementing the functionality of certain embodiments may be stored by file storage subsystem 826 in the storage subsystem 824, or in other machines accessible by the processor(s) 814.

Bus subsystem 812 provides a mechanism for letting the various components and subsystems of the controller 800 communicate with each other as intended. Although bus subsystem 812 is shown schematically as a single bus, alternative embodiments of the bus subsystem may use multiple buses.

The controller 800 can be of varying types including a workstation, server, computing cluster, blade server, server farm, or any other data processing system or computing device. Due to the ever-changing nature of computing devices and networks, the description of the controller 800 depicted in FIG. 8 is intended only as a specific example for purposes of illustrating some embodiments. Many other configurations of the controller 800 are possible having more or fewer components than the controller depicted in FIG. 8.

While the disclosed embodiments have been illustrated and described in considerable detail, it is not the intention to restrict or in any way limit the scope of the appended claims to such detail. It is, of course, not possible to describe every conceivable combination of components or methodologies for purposes of describing the various aspects of the subject matter. Therefore, the disclosure is not limited to the specific details or illustrative examples shown and described. Thus, this disclosure is intended to embrace alterations, modifications, and variations that fall within the scope of the appended claims, which satisfy the statutory subject matter requirements of 35 U.S.C. §101. The above description of specific embodiments has been given by way of example. From the disclosure given, those skilled in the art will not only understand the general inventive concepts and attendant advantages, but will also find apparent various changes and modifications to the structures and methods disclosed. It is sought, therefore, to cover all such changes and modifications as fall within the spirit and scope of the general inventive concepts, as defined by the appended claims, and equivalents thereof.

**REFERENCE NUMERALS**

| | | | |
|---|---|---|---|
| 100 | head-mounted device | 605 | head-mounted device |
| 110 | shell | 620 | auto-darkening device |
| 120 | auto-darkening device | 650 | welding power supply |
| 122 | liquid crystal display (LCD) lens | 660 | controller |
| 124 | selection device | 700 | method |
| 126 | control circuitry | 710 | block |
| 130 | wireless receiver | 720 | block |
| 140 | antenna | 730 | block |
| 400 | arc welding system | 740 | block |
| 410 | power supply | 800 | controller |
| 412 | power conversion unit | 812 | bus subsystem |
| 414 | output inverter | 814 | processor |
| 416 | waveform generator | 816 | network interface subsystem |
| 418 | controller | 820 | interface output device |
| 420 | current feedback ciurcuit | 822 | input device |
| 422 | voltage feedback circuit | 824 | storage subsystem |
| 430 | welding torch | 826 | file storage subsystem |
| 450 | wire feeder | 828 | memory subsystem |
| 460 | wireless transmitter | 830 | main random access memory (RAM) |
| 465 | antenna | | |
| 500 | method | 832 | read only memory (ROM) |
| 510 | block | | |
| 520 | block | W | workpiece |
| 530 | block | E | electrode |
| 540 | block | A | arc |
| 600 | arc welding system | | |

## Claims

1. A head-mounted device (100, 605) for use in arc welding, comprising:
a wireless receiver (130) configured to receive wirelessly transmitted communication signals from an arc welding system (400, 600), where the wirelessly transmitted communication signals represent at least a present welding current setting and a present welding mode setting of the arc welding system (400, 600); and
an auto-darkening device (120, 620) configured to:
sense a presence of an arc (A) produced by the arc welding system (400, 600),
transition from an un-darkened state to a darkened state upon initially sensing the presence of the arc (A),
remain in the darkened state while the arc is present, and
transition from the darkened state to the un-darkened state when the arc (A) is no longer present; and
a selection device (124) configured to automatically select at least a shade and a sensitivity of the auto-darkening device (120, 620) for transitioning to the darkened state in response to at least the present welding current setting and the present welding mode setting of the arc welding system (400, 600) provided to the head-mounted device (100, 605) via the wirelessly transmitted communication signals received by the wireless receiver (130).

2. The head-mounted device (100, 605) of claim 1, wherein the selection device (124) is configured as a programmable look-up-table (LUT) to map input signals or input data, derived from the wirelessly transmitted communication signals and representing the present welding current setting and the present welding mode setting, to output signals or output data representing at least the shade and the sensitivity.

3. The head-mounted device (100, 605) of claim 1 or 2, wherein the selection device (124) is further configured to automatically select at least a reaction time of the auto-darkening (120, 620) device for transitioning to the darkened state in response to at least the present welding current setting and the present welding mode setting of the arc welding system (400, 600) provided to the head-mounted device (100, 605) via the wirelessly transmitted communication signals received by the wireless receiver (130).

4. The head-mounted device (100, 605) of any of the claims 1 to 3, wherein the selection device (124) is further configured to automatically select at least a transition delay time of the auto-darkening device (120, 620) for transitioning back to the un-darkened state in response to at least the present welding current setting and the present welding mode setting of the arc welding system (400, 600) provided to the head-mounted device (120, 620) via the wirelessly transmitted communication signals received by the wireless receiver (130).

5. The head-mounted device (100, 605) of any of the claims 1 to 4, wherein the wirelessly transmitted communication signals include at least one of Wi-Fi® radio frequency spectrum signals, BLUETOOTH® radio frequency spectrum signals, and ZIGBEE® radio frequency spectrum signals, and/or
wherein the wirelessly transmitted communication signals include at least one of infrared spectrum signals and visual spectrum signals, and/or
wherein the wirelessly transmitted communication signals include at least one of ultrasonic spectrum signals and audible spectrum signals.

6. The head-mounted device (100, 605) of any of the claims 1 to 5, wherein a welding mode of the present welding mode setting includes one of a gas metal arc welding (GMAW) mode, a flux-cored arc welding (FCAW) mode, a gas tungsten arc welding (GTAW) mode, and a shielded metal arc welding (SMAW) mode.

7. The head-mounted device (100, 605) of any of the claims 1 to 6, wherein the selection device (124) is integrated into the wireless receiver (130), and/or wherein the wireless receiver (130) is operationally connected to the auto-darkening device (120, 620), and/or
wherein the selection device (124) is integrated into the auto-darkening device (120, 620), and/or wherein the wireless receiver (130) is operationally connected to the auto-darkening device (120, 620).

8. An arc welding system (400, 600), comprising:
a welding power supply (410, 650) including:
a controller (418, 660), and
a wireless transmitter (460) configured to wirelessly transmit communication signals representing at least a present welding current setting and a present welding mode setting of the welding power supply (410, 650) received from the controller (418, 660); and
a head-mounted device (100, 605) including:
a wireless receiver (130) configured to wirelessly receive the communication signals transmitted by the wireless transmitter (460),
an auto-darkening device (120, 620) configured to:
sense a presence of an arc (A) produced by the arc welding system (400, 600),
transition from an un-darkened state to a darkened state upon initially sensing the presence of the arc (A),
remain in the darkened state while the arc (A) is present, and
transition from the darkened state to the un-darkened state when the arc (A) is no longer present, and
a selection device (124) configured to automatically select at least a shade and a sensitivity of the auto-darkening device (120, 620) for transitioning to the darkened state in response to at least the present welding current setting and the present welding mode setting of the welding power supply (410, 650) provided to the head-mounted device (100, 605) via the communication signals wirelessly received by the wireless receiver (130).

9. The arc welding system (400, 600) of claim 8, wherein the selection device (124) is configured as a programmable look-up-table (LUT) to map input signals or input data, derived from the communication signals and representing the present welding current setting and the present welding mode setting, to output signals or output data representing at least the shade and the sensitivity.

10. The arc welding system (400, 600) of claim 8 or 9, wherein the selection device (124) is further configured to automatically select at least a reaction time of the auto-darkening device (120, 620) for transitioning to the darkened state in response to at least the present welding current setting and the present welding mode setting of the welding power supply (410, 650) provided to the head-mounted device (100, 605) via the communication signals wirelessly received by the wireless receiver (130).

11. The arc welding system (400, 600) of any of the claims 8 to 10, wherein the selection device (124) is further configured to automatically select at least a transition delay time of the auto-darkening device (120, 620) for transitioning back to the un-darkened state in response to at least the present welding current setting and the present welding mode setting of the welding power supply (410, 650) provided to the head-mounted device (100, 605) via the communication signals wirelessly received by the wireless receiver (130).

12. The arc welding system (400, 600) of any of the claims 8 to 11, wherein the communication signals include at least one of Wi-Fi® radio frequency spectrum signals, BLUETOOTH® radio frequency spectrum signals, and ZIGBEE® radio frequency spectrum signals and/or
wherein the communication signals include at least one of infrared spectrum signals and visual spectrum signals, and/or
wherein the communication signals include at least one of ultrasonic spectrum signals and audible spectrum signals.

13. The arc welding system (400, 600) of any of the claims 8 to 12, wherein a welding mode of the present welding mode setting includes one of a gas metal arc welding (GMAW) mode, a flux-cored arc welding (FCAW) mode, a gas tungsten arc welding (GTAW) mode, and a shielded metal arc welding (SMAW) mode.

14. The arc welding system (400, 600) of any of the claims 8 to 13, wherein the selection device (124) is integrated into the wireless receiver (130), and/or wherein the wireless receiver (130) is operationally connected to the auto-darkening device (120, 620), and/or
wherein the selection device (124) is integrated into the auto-darkening device (120, 620), and/or wherein the wireless receiver (130) is operationally connected to the auto-darkening device (120, 620).

15. An arc welding system (400, 600), comprising;
a head-mounted device (100, 605) including an auto-darkening device (120, 620) configured to:
sense a presence of an arc (A) produced by the arc welding system (400, 600),
transition from an un-darkened state to a darkened state upon initially sensing the presence of the arc (A),
remain in the darkened state while the arc (A) is present, and
transition from the darkened state to the un-darkened state when the arc (A) is no longer present; and
a welding power supply (400, 600) including:
a controller (418, 660) having a selection device (124), wherein the selection device (124) is configured to map input signals or input data, representing a present welding current setting and a present welding mode setting of the arc welding system (400, 600), to output signals or output data, representing at least an automatically selected shade and sensitivity for the auto-darkening device (120, 620),
a wireless transmitter (460) configured to wirelessly transmit communication signals representing at least the automatically selected shade and sensitivity,
wherein the head-mounted device (100, 605) further includes a wireless receiver (130) configured to wirelessly receive the communication signals transmitted by the wireless transmitter (460) to automatically set at least the automatically selected shade and sensitivity in the auto-darkening device (120, 620) for transitioning to the darkened state.

16. The arc welding system (400, 600) of claim 15, wherein the selection device (124) is configured as a programmable look-up-table (LUT) and includes at least one of an electrically erasable programmable read only memory (EEPROM) device, a field programmable gate array (FPGA) device, a random access memory (RAM) device, and a microprocessor device, and/or
wherein the selection device (124) is further configured to map the input signals or the input data, representing the present welding current setting and the present welding mode setting of the arc welding system, to additional output signals or output data, representing at least an automatically selected reaction time for the auto-darkening device (120, 620) for transitioning to the darkened state, and/or
wherein the selection device (124) is further configured to map the input signals or the input data, representing the present welding current setting and the present welding mode setting of the arc welding system (400, 600), to additional output signals or output data, representing at least an automatically selected transition delay time for the auto-darkening device (120, 620) for transitioning back to the un-darkened state.
